# EUROPÄISCHE PATENTSCHRIFT

(11) **EP 1 244 423 B1**
(45) Veröffentlichungstag und Bekanntmachung des Hinweises auf die Patenterteilung: **04.08.2004**
(21) Anmeldenummer: 00990774.2
(22) Anmeldetag: 15.12.2000
(51) Int. Cl.: A61K 7/32, A61K 7/34, A61K 7/36, A61K 7/38

(54) **ANTITRANSPIRANT-ZUSAMMENSETZUNG ENTHALTEND EINEN WASSERLÖSLICHEN ANTITRANSPIRANT-WIRKSTOFF**
ANTIPERSPIRANT COMPOSITION THAT CONTAINS A WATER-SOLUBLE ANTIPERSPIRANT ACTIVE AGENT
COMPOSITION ANTITRANSPIRANTE COMPRENANT UN AGENT ANTITRANSPIRANT SOLUBLE DANS L'EAU

(30) Priorität: 24.12.1999 DE 19962881
(43) Veröffentlichungstag der Anmeldung: 02.10.2002
(73) Patentinhaber: Henkel Kommanditgesellschaft auf Aktien, 40589 Düsseldorf-Holthausen (DE)
(72) Erfinder: HUNDEIKER, Claudia, 40549 Düsseldorf (DE); BANOWSKI, Bernhard, 40597 Düsseldorf (DE); CLAAS, Marcus, 40723 Hilden (DE)
(86) Internationale Anmeldenummer: PCT/EP2000/012820
(87) Internationale Veröffentlichungsnummer: WO 2001/047488

(56) Entgegenhaltungen:
- US-A- 4 499 069
- US-A- 4 863 721
- US-A- 5 194 262
- US-A- 6 001 341

## Beschreibung

Die Erfindung betrifft cremeartige Antitranspirant-Zubereitungen zum Auftrag wasserlöslicher kosmetischer Antitranspirant-Wirkstoffe auf die Haut.

Antitranspirant-Zusammensetzungen sind der Fachwelt in vielerlei Form bekannt. Als Antitranspirant-Wirkstoffe werden üblicherweise adstringierende Substanzen verwendet, beispielsweise Aluminium- und/oder Zirkoniumsalze. Die Zusammensetzungen sind als Sprays, Roll-on-Präparate, Stifte oder als Cremes im Handel. Derartige Formulierungen werden in Cosmetics: Science and Technology, "Antiperspirants and Deodorants" (Hrsg.: M. S. Balsam und E. G. Sagarin, 1972), Bd. 2, S. 373-416 von S. Plechner beschrieben sowie in Cosmetics Science and Technology Series: "Antiperspirants and Deodorants" (Hrsg.: Karl Laden), 2. Auflage, S. 233 - 258 und S. 327 - 356.

Während der Kosmetikmarkt in den letzten Jahren vorwiegend durch Stift-Präparate beherrscht wurde, erfreuen sich cremeartige Antitranspirant-Zubereitungen zunehmender Beliebtheit. Viele cremeförmige Antitranspirant-Präparate, wie sie in EP 0 310 252 und WO 98/51185 beschrieben sind, werden wasserfrei formuliert. Derartige Präparate hinterlassen beim Anwender ein angenehm trockenes Hautgefühl nach dem Auftragen. Eine effektive Freisetzung der wasserlöslichen Antitranspirant-Wirkstoffe aus solchen Präparaten ist jedoch limitiert (vgl.: Chemistry and Technology of the Cosmetics and Toiletries Industry, Hrsg.: D. F. Williams und W. H. Schmitt, London: Blackie, 1996, 2. Auflage, S. 326) und die Präparate hinterlassen auf der Haut oft kein Frischegfühl. Die wasserfreien Präparate, insbesondere solche auf Basis von flüchtigen Siliconölen haben den Nachteil, daß die dispergierten Wirkstoffe leicht zu sichtbaren Produktrückständen auf Haut und Kleidung führen. Außerdem sind solche Zubereitungen relativ kostspielig, da Ölkomponenten als Wirkstoffträger teuer sind. Unter Druckbelastung bei Applikation kommt es häufig zu einem Ausölen, was die kosmetische Akzeptanz dieser Präparate beim Anwender herabsetzt.

Besser geeignet sind daher emulsionsartige Antitranspirant-Cremes, wie sie beispielsweise in der US 4, 268, 499, WO 97/48373 und WO 98/27946 beschrieben sind. Diese haben jedoch üblicherweise den Nachteil, daß sie nach dem Auftragen auf die Haut ein unangenehmes und langanhaltendes Nässegefühl hinterlassen und von den Anwendern als klebrig oder schmierig empfunden werden. Auf der Haut verbleiben häufig weiße Rückstände dieser Präparate.

Es bestand daher die Aufgabe, eine Antitranspirant-Creme zu entwickeln, die sich als effektiver Träger für wasserlösliche Wirkstoffe eignet, und beim Auftragen auf die Haut keinen schmierigen, feuchten oder klebrigen Eindruck hinterläßt, sondern ein angenehm kühlendes Frischegefühl vermittelt, von der Haut schnell absorbiert wird und sich nach Verdunstung der flüchtigen Bestandteile trocken und samtig anfühlt.

Diese Aufgabe wurde erfindungsgemäß gelöst durch eine wasserhaltige Antitranspirant-Zusammensetzung, gekennzeichnet durch einen Gehalt an:
a) wenigstens zwei teilchenförmigen, wasserunlöslichen Polysacchariden und/oder Polysaccharid-Derivaten
b) wenigstens einem in Wasser gelösten, adstringierenden Antitranspirant-Wirkstoff und
c) wenigstens einer Wachskomponente.

Die erfindungsgemäßen Zubereitungen sind feinteilige Dispersionen der Polysaccharide in einer Wachs/Öl-Phase. Die Wachse bilden mit den restlichen Ölkörpern eine Gelmatrix, die große Mengen Wasser aufnehmen kann. Diese Dispersionen, die durch sehr geringe Mengen an Emulgatoren stabilisiert sind, hinterlassen aufgrund ihres hohen Wassergehaltes bei der Anwendung einen frischen, kühlenden Eindruck. Die teilchenförmige Polysaccharid-Basis erzeugt ein trockenes und samtiges Hautgefühl, hinterläßt keinen schmierigen Rückstand und wird schnell absorbiert. Der Antitranspirant-Wirkstoff ist in der wässrigen Phase gelöst, so daß sich eine deutlich verbesserte Wirkstoffabgabe im Vergleich zu sogenannten "Soft Solids" ergibt. Der Anteil des Wassers an der erfindungsgemäßen Zusammensetzung beträgt 10-70 Gew.-%, vorzugsweise 20 - 60 Gew.-% und insbesondere 30 - 50 Gew.-%. Bevorzugt liegt die erfindungsgemäße Zusammensetzung in Form einer O/W-Dispersion vor, was u. a. durch Verwendung eines geeigneten O/W-Emulgators oder durch geeignete Emulgator-Kombinationen erzielt werden kann. Die Abgabe des Antitranspirant-Wirkstoffes ist hier besonders effizient, da er in der äußeren oder kontinuierlichen Phase der Dispersion enthalten ist.

Die erfindungsgemäßen Dispersionen sind besonders als schweißhemmende Mittel zur Applikation auf der Haut geeignet. In einer bevorzugten Ausführungsform weisen sie bei 21 °C eine Viskosität von wenigstens 50000 mPa·s auf, wobei ein Bereich von 200000 - 1500000 mPa·s bevorzugt ist (Brookfield-RVF, Spindel TE, Heliopath, 4 Upm, 21 °C).

Gegenstand der Erfindung ist auch ein Verfahren zur Herstellung der erfindungsgemäßen Zusammensetzungen, dadurch gekennzeichnet, daß die Wachs- und Ölkomponenten zusammen mit den Emulgatoren und Polysacchariden auf 90 - 95 °C erwärmt und aufgeschmolzen werden, danach das Wasser mit den wasserlöslichen Wirkstoffen zugegeben wird und man die Masse durch Abkühlen auf Raumtemperatur verfestigt.

### 1. Polysaccharide

Als Polysaccharide können erfindungsgemäß natürliche und synthetische Polysaccharide und/oder deren Derivate eingesetzt werden, die teilchen- oder pulverförmig anfallen, und Homo- oder Heteroglykane der üblichen Zuckerbausteine darstellen, und eine Wasserlöslichkeit von weniger als 1 Gew.-% bei 20 °C aufweisen, d. h. daß Mengen von 1 g oder mehr als 1 g des Polysaccharids in 99g Wasser bei 20 °C nicht mehr klar löslich sind. Sie können tierischen (z. B. Chitin, Tunicin), pflanzlichen (z. B. Stärke, Cellulose, Alginsäure), mikrobiellen, bakteriellen oder synthetischen Ursprungs sein. Hierzu gehören die teilchenförmigen Polysaccharide, die beispielsweise in "Encyclopedia of Chemical Technology", Kirk-Othmer, 3. Aufl., 1982, Bd. 3, S. 896-900 und Bd. 15, S. 439-458 beschrieben sind und diejenigen in "Polymers in Nature" (Hrsg.: E. A. MacGregor, C. T. Greenwood), John Wiley and Sons, 1980, Kapitel 6, S. 240-328 sowie in "Industrial Gums - Polysaccharides and their Derivatives" (Hrsg.: R. L. Whistler), 2. Aufl., Academic Press Inc. Erfindungsgemäß können auch Gemische von teilchenförmigen, wasserunlöslichen Polysacchariden oder entsprechenden Derivaten verwendet werden. Synthetische Polysaccharide können aus den üblichen Zuckerbausteinen Glucose, Fructose, Mannose, Galactose, Talose, Gulose, Allose, Idose, Arabinose, Xylose, Lyxose und Ribose bzw. Gemischen davon aufgebaut sein. Ihr Molekulargewicht ist vorzugsweise größer als 5000.

Die Polysaccharide werden in den erfindungsgemäßen Zubereitungen in einer Gesamtmenge von 0,1 - 30 Gew.-%, vorzugsweise 1 - 20 Gew.-% und insbesondere 2 - 10 Gew.-% eingesetzt. Um besonders feinteilige Dispersionen zu erhalten, kann die Verwendung von Polysacchariden mit Teilchengrößen von weniger als 100µm, insbesondere von weniger als 50 µm bevorzugt sein.

Zu den natürlich vorkommenden, teilchenförmig anfallenden Polysacchariden zählen beispielsweise Stärke und Cellulose, die Polykondensationsprodukte der D-Glucose darstellen, sowie Inulin, ein Polykondensat der D-Fructose. Die Molmasse der erfindungsgemäß verwendbaren, hochpolymeren Zucker liegt üblicherweise zwischen 5000 und mehreren Millionen. Sie liegen in den erfindungsgemäßen Zusammensetzungen teilchenförmig vor und verleihen ihnen einen ausgeprägt hautverträglichen Charakter und einen trockenen, samtigen Abrieb.

In einer bevorzugten Ausführungsform enthält die Antitranspirant-Zusammensetzung als teilchenförmige Polysaccharide ein Gemisch aus mindestens einer Stärke und mindestens einer Cellulose.

Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie z. B. Erbsen und Bohnen, Bananen oder dem Mark bestimmter Palmensorten (z. B. Sagopalme) gewonnen werden. Erfindungsgemäß einsetzbar sind natürliche, aus Pflanzen gewonnene Stärken und/oder chemisch oder physikalisch modifizierte Stärken. In einer bevorzugten Ausführungsform enthält die Zusammensetzung mindestens eine hydrophob modifizierte Stärke. Eine Hydrophobierung läßt sich beispielsweise durch Einführung langkettiger oder verzweigter Seitenketten an einer oder mehreren der Hydroxylgruppen der Stärke erreichen. Üblicherweise handelt es sich um Ester, Ether oder Amide der Stärke mit C₁-C₄₀-Resten. Produkte, die Ester oder Ether der Stärke mit Carbonsäuren bzw. Fettalkoholen aus natürlichen Fetten und Ölen darstellen, können bevorzugt sein. Mit einem oder mehreren n-Octenylsuccinatresten veresterte Stärken sind im Sinne der Erfindung bevorzugt. Besonders vorteilhaft ist eine rieselfähige Aluminium-Octenylsuccinat-Stärke, welche z. B. von National Starch unter der Bezeichnung "Dry Flo® Plus" angeboten wird, da sie eine geringe Quellfähigkeit in kaltem Wasser aufweist. Ebenso sind modifizierte, quervernetzte Reisstärken und quervernetzte Distärkephosphorsäureester auf Basis Maisstärke einsetzbar, die von der Dr. Hauser GmbH unter der Bezeichnung Rice® NS, NS TC und unter P.F.A. 11 bzw. Mais PO4® PH"B" vertrieben werden. Stärke oder StärkeDerivate werden in den erfindungsgemäßen Zusammensetzungen in einer Menge von 0,1 - 20 Gew.-%, vorzugsweise 1 - 15 Gew.-% und insbesondere 2 - 10 Gew.-% eingesetzt.

Auch Cellulose, eine ubiquitäre pflanzliche Gerüstsubstanz, ist wasserunlöslich und im Sinne der Erfindung als teilchenförmiges Polysaccharid geeignet. Sie kann in nativer Form, aber auch physikalisch oder chemisch modifiziert eingesetzt werden. Native Cellulose zeichnet sich durch eine besonders gute Hautverträglichkeit aus. In den erfindungsgemäßen Zusammensetzungen bewirkt sie in Kombination mit hydrophob modifizierter Stärke ein besonders trockenes und samtiges Hautgefühl. Zu den chemisch modifizierten, erfindungsgemäß einsetzbaren Cellulosen gehören auch Methylcellulosen mit einem Molekulargewicht und einem Substitutionsgrad, der eine Wasserunlöslichkeit bedingt (typischerweise 0,7 - 1,4 und ca. 2,3 - 3). Weiterhin einsetzbar sind Hydroxyalkylcellulosen, die durch Alkyl- oder Arylalkylgruppen hydrophob modifiziert wurden, wasserunlöslich sind und teilchenförmig anfallen. Unter wasserunlöslich wird eine Löslichkeit von weniger als 1 Gew.-% in Wasser bei 20 °C verstanden. Als Hydroxyalkylcellulosen, die hydrophob modifiziert werden können, kommen beispielsweise Hydroxyethylcellulose, Hydroxypropylcellulose, Hydroxypropylmethylcellulose, Ethylhydroxyethylcellulose und Methylhydroxyethylcellulose in Frage. Die hydrophoben Gruppen sind beispielsweise C₈-C₂₄-Alkyl-, Arylalkyl- oder Alkylaryl-Substituenten. Die Wasserlöslichkeit bzw. -unlöslichkeit hängt vom Molekulargewicht des Polymers und dessen Substitutionsgrad ab. Das Molekulargewicht der Cellulose liegt üblicherweise unter 800000, vorzugsweise bei 20000 - 700000, und der Substitutionsgrad ist so hoch, daß eine Wasserlöslichkeit von weniger als 1 Gew.-% bei 20 °C resultiert, d.h. daß Mengen von 1 g oder mehr als 1g der Cellulose in 99g Wasser bei 20 °C nicht mehr klar löslich sind. Derartige Verbindungen sind in US 4,228,277 und EP 0 412 705 beschrieben. Cetyl-substituierte Hydroxyethylcellulosen, die erfindungsgemäß eingesetzt werden können, werden beispielsweise von der Aqualon Company und von Amerchol hergestellt.

### 2. Wachskomponenten

Unter Wachsen werden natürliche oder künstlich gewonnene Stoffe mit folgenden Eigenschaften verstanden: sie sind von fester bis brüchig harter Konsistenz, grob bis feinkristallin, durchscheinend bis opak, jedoch nicht glasartig, und schmelzen oberhalb von 35 °C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß verwendbar sind natürliche pflanzliche Wachse, wie z. B. Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindunsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen bespielsweise wachsartige Polyalkylenwachse und Polyethylenglycolwachse.

Die Wachskomponente der erfindungsgemäßen wasserhaltigen Antitranspirant-Zusammensetzung wird bevorzugt gewählt aus der Gruppe der Ester aus gesättigten und/oder ungesättigten, verzweigten und/oder unverzweigten Alkancarbonsäuren einer Kettenlänge von 1 - 80 C-Atomen und gesättigten und/oder ungesättigten, verzweigten und/oder unverzweigten Alkoholen einer Kettenlänge von 1 - 80 C-Atomen, aus der Gruppe der Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/oder unverzweigten Alkoholen einer Kettenlänge von 1 - 80 C-Atomen, sowie aus der Gruppe der Lactide langkettiger Hydroxycarbonsäuren, sofern die Wachskomponente oder die Gesamtheit der Wachskomponenten bei Raumtemperatur einen Festkörper darstellen.

Bevorzugt ist, die Wachskomponenten zu wählen aus der Gruppe der
- Ester aus gesättigten, verzweigten Alkancarbonsäuren einer Kettenlänge von 1 - 60 C-Atomen und gesättigten, unverzweigten Alkoholen einer Kettenlänge von 1 - 60 C-Atomen, sofern die Wachskomponente oder die Gesamtheit der Wachskomponenten bei Raumtemperatur einen Festkörper darstellen.
und/oder der
- Ester aus gesättigten, unverzweigten Alkancarbonsäuren einer Kettenlänge von 1 - 60 C-Atomen und gesättigten, verzweigten Alkoholen einer Kettenlänge von 1-60 C-Atomen, sofern die Wachskomponente oder die Gesamtheit der Wachskomponenten bei Raumtemperatur einen Festkörper darstellen.
und/oder der
- Ester aus gesättigten, unverzweigten Alkancarbonsäuren einer Kettenlänge von 1- 60 C-Atomen und gesättigten, unverzweigten Alkoholen einer Kettenlänge von 1 - 60 C-Atomen, sofern die Wachskomponente oder die Gesamtheit der Wachskomponenten bei Raumtemperatur einen Festkörper darstellen.
und/oder der
- Ester aus gesättigten, verzweigten Alkancarbonsäuren einer Kettenlänge von 1 - 60 C-Atomen und gesättigten, verzweigten Alkoholen einer Kettenlänge von 1 - 60 C-Atomen, sofern die Wachskomponente oder die Gesamtheit der Wachskomponenten bei Raumtemperatur einen Festkörper darstellen.

Besonders bevorzugt ist, die Wachskomponenten zu wählen aus der Gruppe der
- Ester aus gesättigten, unverzweigten Alkancarbonsäuren einer Kettenlänge von 14-44 C-Atomen und gesättigten, unverzweigten Alkoholen einer Kettenlänge von 14 - 44 C-Atomen, sofern die Wachskomponente oder die Gesamtheit der Wachskomponenten bei Raumtemperatur einen Festkörper darstellen.

Insbesondere vorteilhaft können die Wachskomponenten aus der Gruppe der C₁₆₋₃₆-Alkylstearate, der C₁₀₋₄₀-Alkylstearate, der C₂₋₄₀Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse wie beispielsweise Stearyltrimethylsilan/Stearylalkohol sind gegebenenfalls vorteilhaft. Das Wachs oder die Wachskomponenten sollten bei 25 °C fest sein, jedoch im Bereich von 35 - 95 °C schmelzen, wobei ein Bereich von 45 - 85 °C bevorzugt ist. Derartige Wachse vermitteln ein besonders angenehmes Hautgefühl.

Eine besonders bevorzugte Ausführungsform der Erfindung enthält als Wachskomponente den Ester aus einem gesättigten, einwertigen C₂₀-C₆₀-Alkohol und einer gesättigten C₈-C₃₀-Monocarbonsäure. Aus dieser Gruppe ist insbesondere ein C₂₀-C₄₀-Alkylstearat bevorzugt, das unter dem Namen Kesterwachs® K82H bekannt ist und von der Firma Koster Keunen Inc. vertrieben wird. Es handelt sich um die synthetische Nachahmung der Monoesterfraktion des Bienenwachses und zeichnet sich durch seine ausgesprochene Härte, seine Ölgelierfahigkeit und seine breite Kompatibiltät mit lipiden Stoffen aus. Es kann als Stabilisator und Konsistenzregulator für W/O- und O/W-Emulsionen verwendet werden. Kesterwachs bietet den Vorteil, daß es auch bei geringen Konzentrationen eine exzellente Ölgelierfähigkeit aufweist und so die Crememasse nicht zu schwer macht und einen samtigen Abrieb ermöglicht. Insbesondere läßt es sich vorteilhaft mit teilchenförmigen Polysacchariden zu feinteiligen Dispersionen verarbeiten, die trotz des hohen Wassergehaltes nicht klebrig sind.

Natürliche, chemisch modifizierte und synthetische Wachse können alleine oder in Kombination eingesetzt werden.

Die Wachskomponenten sind in einer Menge von 0,1 - 40 Gew.-%, bezogen auf die Gesamtzusammensetzung, vorzugsweise 1 - 30 Gew.-% und insbesondere 5 - 15 Gew.-% enthalten.

### 3. Antitranspirant-Wirkstoffe

Als Antitranspirant-Wirkstoffe eignen sich wasserlösliche adstringierende metallische Salze, insbesondere anorganische und organische Salze des Aluminiums, Zirkoniums und Zinks bzw. Mischungen dieser Salze. Erfindungsgemäß verwendbar sind beispielsweise Alaun (KAl(SO₄)₂·12 H₂O), Aluminiumsulfat, Aluminiumlactat, Natrium-Aluminium-Chlorhydroxylactat, Aluminiumchlorhydroxyallantoinat, Aluminiumchlorid, Aluminiumchlorohydrat, Aluminiumchlorohydrex-PEG, Aluminiumchlorohydrex-PG, Aluminiumsulfocarbolat, Aluminiumdichlorohydrat, Aluminiumdichlorohydrex-PEG, Aluminiumdichlorohydrex-PG, Aluminiumsesquichlorohydrate, Aluminiumsesquichlorohydrex-PEG, Aluminiumsesquichlorohydrex-PG, Aluminium-Zirkonium-Chlorohydrate (Aluminium-Zirkonium-Octachlorohydrat, Aluminium-Zirkonium-Pentachlorohydrat, Aluminium-Zirkonium-Tetrachlorohydrat, Aluminium-Zirkonium-Trichlorohydrat), Zinkchlorid, Zinksulfocarbolat, Zinksulfat, Zirkoniumchlorohydrat und Aluminium-Zirkonium-Chlorohydrat-Glycin-Komplexe, wie beispielsweise Aluminium-Zirkonium-Octachlorohydrex-Glycin, Aluminium-Zirkonium-Pentachlorohydrex-Glycin, Aluminium-Zirkonium-Tetrachlorohydrex-Glycin, Aluminium-Zirkonium-Trichlorohydrex-Glycin. Erfindungsgemäß wird unter Wasserlöslichkeit eine Löslichkeit von wenigstens 5 Gew.-% bei 20 °C verstanden, d. h. daß wenigstens 5 g des Antitranspirant-Wirkstoffes in 95g Wasser bei 20 °C löslich sind. Die Antitranspirant-Wirkstoffe werden bei wässrigen Applikationen als wässrige Lösungen eingesetzt. Sie sind in den erfindungsgemäßen Zusammensetzungen in einer Menge von 1 - 40 Gew.-%, vorzugsweise 5 - 30 Gew.-% und insbesondere 10 - 20 Gew.-% enthalten (bezogen auf die Menge der Aktivsubstanz in der Gesamtzusammensetzung). In einer bevorzugten Ausführungsform enthält die Zusammensetzung ein adstringierendes Aluminiumsalz, insbesondere Aluminiumchlorohydrat, das beispielsweise pulverförmig als Micro Dry® Ultrafine von Reheis, in Form einer wäßrigen Lösung als Locron® L von Clariant, als Chlorhydrol® sowie in aktivierter Form als Reach® 501 von Reheis vertrieben wird. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G im Handel sind, kann erfindungsgemäß besonders vorteilhaft sein.

### 4. Emulgatoren

Erfindungsgemäß geeignet sind insbesondere nicht-ionische Emulgatoren, die sich gegenüber anionischen Emulgatoren durch eine bessere Hautverträglichkeit auszeichnen. Um besonders feinteilige Dispersionen zu erhalten, ist es vorteilhaft, eine Kombination von nicht-ionischen Emulgatoren einzusetzen. Zu den geeigneten nicht-ionischen Emulgatoren gehören beispielsweise:
(1) Anlagerungsprodukte von 2 - 50 Mol Ethylenoxid und/oder 0 - 20 Mol Propylenoxid an lineare Fettalkohole mit 8 - 40 C-Atomen, an Fettsäuren mit 12 - 40 C-Atomen und an Alkylphenole mit 8 - 15 C-Atomen in der Alkylgruppe.
(2) C_{12/18}-Fettsäuremono- und -diester von Anlagerungsprodukten von 1 - 50 Mol Ethylenoxid an Glycerin.
(3) Glycerinmono- und -diester und Sorbitanmono- und -diester von gesättigten und ungesättigten Fettsäuren mit 6 - 22 Kohlenstoffatomen und deren Ethylenoxidanlagerungsprodukte.
(4) Alkylmono- und -oligoglycoside mit 8 - 22 Kohlenstoffatomen im Alkylrest und deren ethoxylierte Analoga.
(5) Anlagerungsprodukte von 15 - 60 Mol Ethylenoxid an Ricinusöl und/oder gehärtetes Ricinusöl.
(6) Polyol- und insbesondere Polyglycerinester, wie z. B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen.
(7) Anlagerungsprodukte von 2 - 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose).
(9) Wollwachsalkohole.
(10) Polysiloxan-Polyalkyl-Polyether-Copolymere bzw. entsprechende Derivate.
(11) Mischester aus Pentaerythrit, Fettsäuren, Citronensäure und Fettalkohol gemäß **DE 1165574** und/oder Mischester von Fettsäuren mit 6-22 Kohlenstoffatomen, Methylglucose und Polyolen, vorzugsweise Glycerin oder Polyglycerin.
(12) Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 - 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® zur Verfügung stehen, enthalten eine glucosidisch gebundene C₈-C₁₆-Alkylgrupge an einem Oligoglucosidrest, dessen mittlerer Oligomerisationsgrad bei 1 - 2 liegt. Auch die vom Glucamin abgeleiteten Acylglucamide sind als nicht-ionische Emulgatoren geeignet.

Erfindungsgemäß kann es auch vorteilhaft sein, handelsübliche Emulgatorgemische, wie z. B. Plantacare® PS 10, ein Fettalkoholethersulfat/Alkylpolyglucosid-Gemisch, oder Cutina® KD 16 V, ein C₁₆-C₁₈-Fettsäuremono/Diglycerid/Kaliumstearat-Gemisch einzusetzen.

### 4.1 O/W-Emulgatoren

In einer bevorzugten Ausführungsform enthält die Zusammensetzung mindestens einen nicht-ionischen Emulgator mit einem HLB-Wert von 8 - 18. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert erfindungsgemäß nach folgender Formel berechnet: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist. Der O/W-Emulgator wird in einer Menge von 0,1 - 10 Gew.-%, vorzugsweise 0,1 - 5 Gew.-% und insbesondere 0,5 - 3 Gew.-% bezogen auf die Gesamtzusammensetzung eingesetzt. Auch der Einsatz eines Gemisches von O/W-Emulgatoren kann vorteilhaft sein.

Zu dieser Gruppe von Emulgatoren gehören u. a. Anlagerungsprodukte von 10 - 50 Mol Ethylenoxid an Fettalkohole, Fettsäuren, Fettsäurealkanolamide, Fettsäuremonoglyceride, Sorbitanfettsäureester, Methylglucosid-Fettsäureester oder Polyglycolether-modifizierte Polysiloxane.

Vorzugsweise werden Alkoholethoxylate der Formel R¹O(CH₂CH₂O)ₙH eingesetzt, die je nach Herkunft des Alkohols als Fettalkoholethoxylate oder als Oxoalkoholethoxylate bezeichnet werden; R¹ steht für einen linearen oder verzweigten Alkyl- und/oder Alkenylrest mit 6 - 22 Kohlenstoffatomen und n für Zahlen von 10 - 50. Typische Vertreter sind die Addukte von durchschnittlich 10 - 50, vorzugsweise 10 - 30 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Auch Addukte von 10 - 40 Mol Ethylenoxid an technische Fettalkohole mit 12 - 18 Kohlenstoffatomen, wie beispielsweise Kokos-, Palm-, Palmkern- oder Talgfettalkohol sind geeignet.

### 4.2 W/O-Emulgatoren

Eine weitere bevorzugte Ausführungsform der Erfindung ist dadurch gekennzeichnet, daß sie mindestens einen lipophilen Coemulgator enthält. Die Verwendung von Coemulgatoren trägt zur Ausbildung besonders feinteiliger Dispersionen bei. Als lipophile Coemulgatoren eignen sich prinzipiell Emulgatoren mit einem HLB-Wert von 1-10. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Addukte läßt sich der HLB-Wert, wie bereits erwähnt, auch berechnen. Als Coemulgator verwendbar sind beispielsweise:
(1) Gesättigte Alkohole mit 8 - 24 C-Atomen, insbesondere mit 16 - 22 C-Atomen, z. B. Cetylalkohol, Stearylalkohol, Arachidylalkohol oder Behenylalkohol oder Gemische dieser Alkohole, wie sie bei der technischen Hydrierung von pflanzlichen und tierischen Fettsäuren erhalten werden.
(2) Ethoxylierte Alkohole und Carbonsäuren mit 8 - 24 C-Atomen, insbesondere mit 16 - 22 C-Atomen, die einen HLB-Wert von 1 - 8 aufweisen.
(3) Propoxylierte Alkohole und Carbonsäuren mit 8 - 24 C-Atomen, insbesondere mit 16 - 22 C-Atomen.
(4) Partialester aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind einsetzbar.
(5) Polyglycerinester gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Verersterungsgrad von 1 - 10, vorzugsweise 1 - 5.
(6) Mono- und/oder Polyglycerinether gesättigter und/oder ungesättigter, verzweigter und/oder unverzweigter Alkohole einer Kettenlänge von 8 - 30, insbesondere 12-18 C-Atomen, mit bis zu 10 Glycerineinheiten, vorzugsweise bis zu 3 Glycerineinheiten und einem Veretherungsgrad von 1 bis 10, vorzugsweise 1 bis 5.
(7) Propylenglycolester gesättigter und/oder ungesättigter, verzweigter und/oder unverzweigter Alkancarbonsäuren einer Kettenlänge von 8 - 24, insbesondere 12 - 18 C-Atomen.
(8) Methylglucose-Ester gesättigter und/oder ungesättigter, verzweigter und/oder unverzweigter Alkancarbonsäuren einer Kettenlänge von 8 - 24, insbesondere 12 - 18 C-Atomen.
(9) Polyglycerin-Methylglucose-Ester gesättigter und/oder ungesättigter, verzweigter und/oder unverzweigter Alkancarbonsäuren einer Kettenlänge von 8 - 24, insbesondere 12 - 18 C-Atomen.

Es kann erfindungsgemäß von Vorteil sein, daß niedrig ethoxylierte (3 - 5 EO) und/oder propoxylierte Produkte Verwendung finden, beispielsweise polyethoxyliertes hydrogeniertes oder nichthydrogeniertes Ricinusöl oder ethoxyliertes Cholesterin.

Besonders vorteilhaft aus der Gruppe der W/O-Emulgatoren sind Glyceryllanolat, Glycerylmonostearat, Glycerylmonoisostearat, Glycerylmonomyristat, Glycerylmonooleat, Diglycerylmonostearat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearylether (Steareth-2), Glycerylmonolaurat, Glycerylmonocarpinat, Glycerylmonocaprylat, Glycerylsorbitanstearat, Polyglyceryl-4-Isostearat, Polyglyceryl-2-sesquiisostearat, PEG-7- hydrogeniertes Ricinusöl, PEG-40-Sorbitanperisostearat, Isostearyldiglycerylsuccinat und PEG-5-Cholesterylether.

In den erfindungsgemäßen Zusammensetzungen liegt der W/O-Emulgator vorteilhafterweise in einer Konzentration von 0,1-10 Gew.-%, vorzugsweise 0,1 - 5 Gew.-% und insbesondere 0,5 - 3 Gew.-% bezogen auf das Gesamtgewicht der Zusammensetzung vor.

### 5. Weitere Lipide und Pflegekomponenten

### 5.1 Silicone

Als Ölkörper geeignet sind insbesondere Silikonöle. Zu diesen zählen z. B. Dialkyl- und Alkylarylsiloxane, wie beispielsweise Dimethylpolysiloxan und Methylphenylpolysiloxan, sowie deren alkoxylierte und quaternierte Analoga.

Eine bevorzugte Ausführungsform der Zusammensetzung enthält wenigstens eine flüchtige Silicon-Komponente. Die geeigneten flüchtigen Silikone können linear, verzweigt oder cyclisch sein, sind in Todd et al., "Volatile Silicone Fluids for Cosmetics", Cosmetics and Toiletries, S. 27 - 32 (1976) beschrieben und sollen Teil der Offenbarung der vorliegenden Anmeldung sein. Bevorzugt im Sinne der Erfindung sind Silicone mit 3 - 7, insbesondere 4 - 6 Siliciumatomen. Insbesondere bevorzugt sind cyclische Polydimethylsiloxane, wie beispielsweise Decamethylcyclopentasiloxan, Octamethylcyclotetrasiloxan oder Hexamethylcyclotrisiloxan, die unter der Bezeichnung Cyclomethicone bekannt sind. Kommerziell erhältlich sind diese von G. E. Silicones als Cyclomethicone D-4 und D-5, von Dow Corning Corp. als Dow Corning® 344, 345 und 244, 245, 246, von General Electric Co. als GE® 7207 und 7158. Unter den linearen flüchtigen Silikonen sind diejenigen mit 1 - 7 und vorzugsweise solche mit 2 - 3 Siliciumatomen bevorzugt. Die flüchtigen Silicone sind in einer Menge von 0,1 - 30 Gew.-%, vorzugsweise 1 - 20 Gew.-% und insbesondere 3 - 10 Gew.-% enthalten.

Vorteilhafterweise kann die Zusammensetzung nicht-flüchtige lineare Silikonöle enthalten, insbesondere solche, die eine Viskosität von weniger als 500 mm²/s bei 25 °C aufweisen (Brookfield: Model RVDV II+, Spindel 4, 20 Upm). Entsprechende Polyalkylsiloxane, Polyalkylarylsiloxane und Polyethersiloxan-Copolymere sind in Cosmetics: Science and Technology, Hrsg.: M. Balsam und E. Sagarin, Bd. 1, 1972, S. 27-104, in US 4,202,879 und US 5,069,897 beschrieben und sollen Teil der Offenbarung der vorliegenden Anmeldung sein. Die nicht-flüchtigen Silicone sind in einer Menge von 0,1 - 15 Gew.-%, vorzugsweise 1 - 10 Gew.-% und insbesondere 1 - 5 Gew.-% enthalten.

Eine besonders bevorzugte Ausführungsform ist dadurch gekennzeichnet, daß sie
a) 0,1 - 25 Gew.-% einer teilchenförmigen Stärke; b) 0,1 - 25 Gew.-% einer teilchenförmigen Cellulose; c) 0,1 - 15 Gew.-% eines Esters eines Fettalkohols mit einer Fettsäure;
d) 0,1 - 40 Gew.-% eines adstringierenden Antitranspirantsalzes; e) 0,1 - 20 Gew.-% einer flüchtigen Silikonverbindung; f) 0,1 - 5 Gew.-% eines Emulgators mit einem HLB-Wert von 8 - 18 und g) 10 - 65 Gew.-% Wasser enthält.

### 5.2 Natürliche und synthetische Öle und Fette

Neben den Wachsen, die als zwingende Komponente in den erfindungsgemäßen Zusammensetzungen enthalten sind, ist eine Reihe weiterer Öl- und Fettkomponenten vorteilhaft. Hierzu zählen u. a. Fettsäure- und Fettalkoholester, insbesondere Mono-, Di- und Triglyceride sowie die Ester von Fettsäuren mit Alkoholen mit 1 - 24 C-Atomen, sofern diese nicht unter die Gruppe der Wachse fallen. Hierzu gehören u.a. die Produkte der Veresterung von Fettsäuren mit 6 - 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder von Aldehyden aus der Roelen'schen Oxosynthese sowie als Monomerfraktion bei der Dimerisierung von ungesättigten Fettalkoholen anfallen. Besonders geeignet kann die Verwendung natürlicher Fett und Öle wie z. B. Rindertalg, Erdnußöl, Rüböl, Baumwollsaatöl, Sojaöl, Sonnenblumenöl, Palmöl, Palmkernöl, Leinöl, Mandelöl, Rizinusöl, Maisöl, Olivenöl, Rapsöl, Sesamöl, Kakaobutter und Kokosfett und dergleichen sein. Auch die hydrierten oder gehärteten Öle, z. B. hydriertes Sojaöl, Rizinusöl und Erdnußöl können verwendet werden.

Vorteilhaft in der Gruppe der Fettsäureester sind ferner pflegende und rückfettende Komponenten, wie z. B. Isopropylmyristat, Isopropylpalmitat, Isopropylstearat und -isostearat, Isopropyloleat, n-Butylstearat, n-Hexyllaurat, n-Decyloleat, Isooctylstearat, Isononylstearat, Isocetylstearat, Isononylisononanoat, Isotridecylisononanoat, Cetearylisononanot, Glyceryltriisostearin, 2-Ethylhexylpalmitat und -isostearat, 2-Ethylhexyllaurat, 2-Hexyldecylstearat, 2-Octyldodecylpalmitat, Oleyloleat, Oleylerucat, Erucyloleat, Erucylerucat, 2-Ethylhexylisostearat, 2-Ethylhexylcocoat, C₁₂₋₁₅-Alkylbenzoat, C₁₂-C₁₅-Alkoholactat, Capryl-Caprinsäuretriglycerid, Ethylenglycoldioleat und -dipalmitat, Di-(2-Ethylhexyl)-adipat, Di-n-butyl-adipat, Dicetyl-/stearyl-adipat, Di-2-ethylhexylsuccinat, Dicetylsuccinat, Diethyl-/Di-n-butyl/Dioctylsebacat, 1,2-Propylenglycoldistearat, Butyloctansäure-2-butyloctanoat, Diisotridecylacetat, sowie synthetische, halbsynthetische und natürliche Gemische solcher Ester, z. B. Jojobaöl.

In einer besonders bevorzugten Ausführungsform enthält die Zusammensetzung zusätzlich wenigstens einen Glycerinester mit einem Schmelzpunkt im Bereich von 28 - 45°C. Erfindungsgemäß können hierfür Mono-, Di- oder Triglyceride sowie beliebige Mischungen der Glyceride eingesetzt werden. Diese Glycerinester ermöglichen eine Konsistenzoptimierung des Produktes und eine Minimierung der sichtbaren Rückstände auf der Haut. Besonders bevorzugt ist Novata® AB, ein Gemisch aus C₁₂-C₁₈-Mono-, Di- und Triglyceriden, welches im Bereich von 30 - 32 °C schmilzt.

### 5.3 Fettalkohole mit 8 - 22 C-Atomen

Die eingesetzten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Hexyldecanol, Octyldodecanol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol sowie deren Guerbet-Alkohole. Die Fettalkohole werden üblicherweise aus den Estern der Fettsäuren durch Reduktion gewonnen. Erfindungsgemäß einsetzbar sind ebenfalls Fettalkoholschnitte, die durch Reduktion natürlich vorkommender Fette und Öle, wie z. B. Rindertalg, Erdnußöl, Rüböl, Baumwollsaatöl, Sojaöl, Sonnenblumenöl, Palmkemöl, Leinöl, Rizinusöl, Maisöl, Rapsöl, Sesamöl, Kakaobutter und Kokosfett entstehen. Es können aber auch synthetische Alkohole, z. B. die linearen, geradzahligen Fettalkohole der Ziegler-Synthese (Alfole® ) oder die teilweise verzweigten Alkohole aus der Oxosynthese (Dobanole® ) verwendet werden.

Ferner können natürliche und synthetische Kohlenwasserstoffe wie beispielsweise Paraffinöle, Isohexadecan, Isoeicosan oder Polydecene, die beispielsweise unter der Bezeichnung Emery® 3004, 3006, 3010, unter der Bezeichnung Ethylflo® von Albernarle oder Nexbase® 2004G von Nestle erhältlich sind, sowie 1,3-Di-(2-ethyl-hexyl)-cyclohexan (Cetiol® S), Di-n-alkylether mit insgesamt 12 - 24 C-Atomen wie z. B. Di-n-octylether, Di-(2-ethylhexyl)-ether, Laurylmethylether oder Octylbutylether und/oder Fettsäuremonooder -diester des Sorbitans oder Methylglucosids, jeweils von Fettsäuren mit 8 - 22 C-Atomen eingesetzt werden.

### 6. Geruchsabsorber und Pigmente

Eine bevorzugte Ausführungsform der Zusammensetzung enthält zusätzlich wenigstens ein Silicat. Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Sie werden in einer Menge von 0,1 - 20 Gew.-%, vorzugsweise 1 - 10 Gew.-% und insbesondere 1 - 5 Gew.-% eingesetzt. Weitere, vorteilhafte Geruchsabsorber sind beispielsweise Zeolithe, Zinkricinoleat, Cyclodextrine, bestimmte Metalloxide, wie z. B. Aluminiumoxid sowie Chlorophyll. Ferner können Pigmente, z. B. Titandioxid, eingesetzt werden, um die kosmetische Akzeptanz des Präparates beim Anwender zu unterstützen.

### 7. Weitere Wirkstoffe/fakultative Komponenten

In die erfindungsgemäßen Zusammensetzungen können vorteilhafterweise zusätzlich die üblichen Bestandteile kosmetischer Präparate eingearbeitet werden, z. B. Stabilisatoren, Verdickungsmittel, Feuchthaltemittel, Hilfs- und Zusatzstoffe wie Farbstoffe und Parfümöle, Nanosphären, desodorierende Wirkstoffe, Konservierungs- und Lichtschutzmittel, Antioxidantien, Enzyme sowie Pflegestoffe. Diese sind vorzugsweise in einer Menge von 0,1 - 20 Gew.-% in der Zubereitung enthalten.

### 7.1 Feuchthaltemittel/Hautbefeuchtungsmittel

Die Zusammensetzung enthält vorzugsweise auch ein Feuchhaltemittel oder eine Kombination aus Feuchhaltemitteln, die der Feuchtigkeitsregulierung der Haut dienen. Erfindunsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer.

Die Menge der Feuchthaltemittel in den erfindungsgemäßen Zusammensetzungen beträgt 0,1 - 15 Gew.-%, vorzugsweise 1 - 10 Gew.-% und insbesondere 2 - 8 Gew.-%, bezogen auf das Gesamtgewicht der Zusammensetzung.

### 7.2 Desodorierende Wirkstoffe

Erfindungsgemäß einsetzbar sind Geruchsüberdecker (z. B. Parfüm), Geruchsabsorber oder -löscher (*vide supra*), desodorierend wirkende Ionenaustauscher, keimhemmende Mittel sowie Enzyminhibitoren oder eine Kombination der genannten Wirkstoffe.

Als keimhemmende oder antimikrobielle Wirkstoffe erfindungsgemäß einsetzbar sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Cloflucarban, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, Glycerinmonoalkylether, Carbonsäureester des Mono-, Di- und Triglycerins (z. B. Glycerinmonolaurat, Diglycerinmonocaprinat), Fettsäuren sowie Pflanzenextrakte (z. B. grüner Tee und Bestandteile des Lindenblütenöls) einsetzbar. Zu den Enzyminhibitoren gehören Stoffe, welche die für die Schweißzersetzung verantwortlichen Enzyme, insbesondere die esterspaltenden Lipasen, hemmen, z. B. Zitronensäuretriethylester oder Zinkglycinat.

Die Menge der desodorierenden Wirkstoffe in den erfindungsgemäßen Zusammensetzungen beträgt 0,001 - 10 Gew.-%, vorzugsweise 0,01 - 5 Gew.-% und insbesondere 0.1 - 3 Gew.-%, bezogen auf das Gesamtgewicht der Zusammensetzung.

### 7.3 Konservierungsstoffe

Den erfindungsgemäßen Zusammensetzungen können fakultativ auch die üblichen Konservierungsstoffe zugesetzt werden, welche die Aufgabe haben, eine Kontamination des Produktes durch Mikroorganismen zu verhindern. Damit haben zahlreiche Konservierungsstoffe zwangsläufig auch desodorierende Eigenschaften, so daß einige Stoffe beiden Gruppen angehören. Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z. B. Methyl-, Ethyl-, Propyl-, Isopropyl-, Butyl- Isobutyl-, Isodecyl-, Phenyl-, Phenoxyethyl- und Benzylparaben, Hexamidinparaben und -diparaben, Natrium- und Kaliumparaben, Natrium- und Kaliummethylparaben, Kaliumbutylparaben, Natrium- und Kaliumpropylparaben), Alkohole und deren Salze (z. B. Ethanol, Propanol, Isopropanol, Benzylalkohol, Phenethylalkohol, Phenol, Kaliumphenolat, Phenoxyethanol, Phenoxyisopropanol, o-Phenylphenol,), Guajacol und dessen Derivate, Chlorhexidin und dessen Derivate (z. B. Chlorhexidindiacetat, -digluconat, und - dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylharnstoff, Imidazolidinylharnstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquaternium-42, Quatemium-8, Quaternium-14, Quaternium-15), Carbamate (z. B. Iodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Triclosan, Zinkpyrithion sowie zahlreiche Peptid-Antibiotika (z. B. Nisin).

Die Menge der Konservierungsmittel in den erfindungsgemäßen Zusammensetzungen beträgt 0,001 -10 Gew.-%, vorzugsweise 0,01- 5 Gew.-% und insbesondere 0,1 - 3 Gew.-%, bezogen auf das Gesamtgewicht der Zusammensetzung.

### 7.4 Antioxidantien

Besonders vorteilhaft werden die Antioxidantien gewählt aus der Gruppe, bestehend aus Aminosäuren (z. B. Glycin, Histidin, Tyrosin, Tryptophan) und deren Derivate, Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol - µmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin, und deren Derivate, ungesättigte Fettsäuren und deren Derivate (z. B. gamma-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -acetat, -stearat, -dipalmitat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth50, Tocophersolan), Vitamin A und Derivate (z.B. Vitamin-A-Palmitat), das Coniferylben-Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und -salicylat, Butylhydroxytoluol, Butylhydroxyanisol, Nordihydroguajakaharzsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.

Die Gesamtmenge der Antioxidantien in den erfindungsgemäßen Zusammensetzungen beträgt 0,001 - 10 Gew.-%, vorzugsweise 0,05-5 Gew.-% und insbesondere 0,1 - 2 Gew.-%, bezogen auf das Gesamtgewicht der Zubereitung.

Als lipophile, öllösliche Antioxidantien aus dieser Gruppe sind Tocopherol und dessen Derivate, Gallussäureester, Flavonoide und Carotinoide sowie Butylhydroxytoluol/anisol bevorzugt. Weiterhin können öllösliche UV-Filtersubstanzen sowie alle bekannten in der Lipidschmelze löslichen kosmetischen oder dermatologischen Wirkstoffe enthalten sein. Als öllösliche Deodorantien und Konservierungsmittel sind Triethylcitrat bzw. Triclosan besonders geeignet.

Im Sinne der vorliegenden Erfindung sind unter den wasserlöslichen Antioxidantien diejenigen bevorzugt, die nicht nur die Bestandteile der Formulierung, sondern auch die Haut, vor oxidativer Beanspruchung schützen können. Hierzu zählen beispielsweise verschiedene Aminosäuren, z. B. Tyrosin und Cystein, und deren Derivate sowie Gerbstoffe, insbesondere solche pflanzlichen Ursprungs.

Die erfindungsgemäßen Zusammensetzungen können weitere kosmetisch und dermatologisch wirksame Stoffe enthalten, wie z. B. Biotin, Pantothensäure, Sebostatika, Anti-Akne-Wirkstoffe sowie Keratolytika. Auch entzündungshemmende und die Heilung fördernde Stoffe wie z. B. Allantoin, Panthenol und verschiedene Pflanzenextrakte und Proteinhydrolysate können vorteilhaft sein.

### Erfindungsgemäße Beispiele

Die Emulgatoren, Öle und Wachskomponenten wurden zusammen auf 95°C bis zur klaren Schmelze erwärmt. In diese heiße Fettphase wurde das Gemisch aus Wasser, wasserlöslichen Wirkstoffen, Polyolen und dem Aluminiumchlorohydrat unter Rühren bei 80°C eingearbeitet. Es bildete sich eine Emulsion, die unter Rühren abgekühlt wurde. Anschließend wurde das Parfümöl und ggf. temperaturempfindliche Wirkstoffe zugegeben.
Die Mengenangaben in nachfolgenden Beispielen beziehen sich, soweit nicht anders angegeben, auf Gew.-% Aktivsubstanz der Gesamtzusammensetzung.

### Beispiel 1

| | |
|---|---|
| 1 | Gew.-% Behenylalkohol + 10 EO (z. B. Mergital® B10) |
| 5 | Gew.-% C₂₀-C₄₀-Alkylstearat (z. B. Kesterwachs® K82H) |
| 1,5 | Gew.-%Triglycerindiisostearat (z. B. Lameform® TGI) |
| 3 | Gew.-% Hexyldecyllaurat / Hexyldecanol (z. B. Cetiol® PGL) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 5 | Gew.-% Aluminium-Stärke-Octenylsuccinat (z. B. Dry Flo® Plus) |
| 5 | Gew.-% Cellulose (z. B. Vitacel® L 600-20 FCC) |
| 4 | Gew.-% 1,3-Butylenglycol |
| 40 | Gew.-% Aluminiumchlorohydrat (50%-ige Lsg. in Wasser, z. B. Chlorhydrol® ) |
| 1 | Gew.-% Parfümöl |
| 0,2 | Gew.-% Methylparaben |
| 0,2 | Gew.-% Tocopherylacetat |
| ad 100 Gew.-% Wasser VE | |

### Beispiel 2

| | |
|---|---|
| 1 | Gew.-% Behenylalkohol + 10 EO (z. B. Mergital® B10) |
| 5 | Gew.-% C₂₀-C₄₀-Alkylstearat (Kesterwachs® K82H) |
| 1,5 | Gew.-%Triglycerindiisostearat (z. B. Lameform® TGI) |
| 3 | Gew.-% Hexyldecyllaurat / Hexyldecanol (z. B. Cetiol® PGL) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 5 | Gew.-% Tapioca |
| 5 | Gew.-% Cellulose (z. B. Vitacel® 600/20 FCC) |
| 4 | Gew.-% 1,3-Butylenglycol |
| 40 | Gew.-% Aluminiumchlorohydrat (50%-ige Lsg. in Wasser) |
| 1 | Gew.-% Parfümöl |
| 0,02 | Gew.-% Butylhydroxytoluol |
| ad 100 Gew.-% Wasser VE | |

### Beispiel 3

| | |
|---|---|
| 1 | Gew.-% Behenylalkohol + 10 EO (z. B. Mergital® B10) |
| 5 | Gew.-% C₂₀-C₄₀-Alkylstearat (Kesterwachs® K82H) |
| 1,5 | Gew.-%Triglycerindiisostearat (z. B. Lameform® TGI) |
| 3 | Gew.-% Hexyldecyllaurat / Hexyldecanol (z. B. Cetiol® PGL) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 5 | Gew.-% Aluminium-Stärke-Octenylsuccinat (z. B. Dry Flo® Plus) |
| 5 | Gew.-% Tapioca |
| 2 | Gew.-% Talkum |
| 4 | Gew.-% 1,3-Butylenglycol |
| 40 | Gew.-% Aluminiumchlorohydrat (50%-ige Lsg. in Wasser) |
| 1 | Gew.-% Parfümöl |
| 0,05 | Gew.-% Gallussäurepropylester |
| ad 100 Gew.-% Wasser VE | |

### Beispiel 4

| | |
|---|---|
| 1 | Gew.-% Behenylalkohol + 10 EO (z. B. Mergital® B10) |
| 1 | Gew.-% Cetylstearylalkohol + 20EO (z. B. Eumulgin® B2) |
| 5 | Gew.-% C₂₀-C₄₀-Alkylstearat (Kesterwachs® K82H) |
| 1,5 | Gew.-%Triglycerindiisostearat (z. B. Lameform® TGI) |
| 3 | Gew.-% Hexyldecyllaurat / Hexyldecanol (z. B. Cetiol® PGL) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 5 | Gew.-% Aluminium-Stärke-Octenylsuccinat (z. B. Dry Flo® Plus) |
| 5 | Gew.-% Cellulose (z. B. Vitacel® L 600-20 FCC) |
| 4 | Gew.-% 1,3-Butylenglycol |
| 48 | Gew.-% Aluminiumchlorohydrat (50%-ige Lsg. in Wasser) |
| 1 | Gew.-% Parfümöl |
| 0,5 | Gew.-% Phenoxyethanol |
| ad 100 Gew.-% Wasser VE | |

### Beispiel 5

| | |
|---|---|
| 1 | Gew.-% Cetylstearylalkohol + 20 EO (z. B. Eumulgin® B2) |
| 5 | Gew.-% C₂₀-C₄₀-Alkylstearat (z. B. Kesterwachs® K82H) |
| 1,5 | Gew.-%Triglycerindiisostearat (z. B. Lameform® TGI) |
| 3 | Gew.-% Hexyldecyllaurat / Hexyldecanol (z. B. Cetiol® PGL) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 5 | Gew.-% Aluminium-Stärke-Octenylsuccinat (z. B. Dry Flo® Plus) |
| 5 | Gew.-% Cellulose (z. B. Vitacel® L 600-20 FCC) |
| 4 | Gew.-% 1,3-Butylenglycol |
| 50 | Gew.-% Aluminium-Zirkonium-Tetrachlorohydrex-Glycin (z. B. Rezal® 36G, 35%-ige Lösung in Wasser) |
| 1 | Gew.-% Parfümöl |
| 0,2 | Gew.-% Tocopherylacetat |
| ad 100 Gew.-% Wasser VE | |

### Beispiel 6

| | |
|---|---|
| 1 | Gew.-% Cetylstearylalkohol + 20 EO (z. B. Eumulgin® B2) |
| 5 | Gew.-% C₂₀-C₄₀-Alkylstearat (z. B. Kesterwachs® K82H) |
| 1 | Gew.-% Glycerylmonooleat (z. B. Monomuls® 90-O-18) |
| 3 | Gew.-% Hexyldecyllaurat / Hexyldecanol (z. B. Cetiol® PGL) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 5 | Gew.-% Aluminium-Stärke-Octenylsuccinat (z. B. Dry Flo® Plus) |
| 5 | Gew.-% Cellulose (z. B. Vitacel® L 600-20 FCC) |
| 4 | Gew.-% 1,3-Butylenglycol |
| 40 | Gew.-% Aluminiumchlorohydrat (50%-ige Lsg. in Wasser) |
| 0,2 | Gew.-% Tocopherylacetat |
| 0,1 | Gew.-% Triclosan |
| 1 | Gew.-% Parfümöl |
| ad 100 Gew.-% Wasser VE | |

### Beispiel 7

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Stearyl/Behenylalkohol (z. B. Stenol® 1822 A) |
| 3,0 | Gew.-% Cocoglycerid (z. B. Novata® AB) |
| 8,0 | Gew.-% Decamethylcyclopentasiloxan (z. B. Dow Corning® 245-Fluid) |
| 1,5 | Gew.-% Polydimethylsiloxan (z. B. Baysilon® M350) |
| 1,5 | Gew.-% Aluminium-Stärke-Octenylsuccinat (z. B. Dry Flo® Plus) |
| 0,5 | Gew.-% modifizierte Reisstärke (z. B. P.F.A. 11) |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 8

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Behenylalkohol (z. B. Lanette® 22) |
| 3,0 | Gew.-% Cocoglycerid (z. B. Novata® AB) |
| 8,0 | Gew.-% Decamethylcyclopentasiloxan (z. B. Dow Corning® 345-Fluid) |
| 1,5 | Gew.-% Polydimethylsiloxan (z. B. Baysilon® M350) |
| 1,0 | Gew.-% Aluminium-Stärke-Octenylsuccinat (z. B. Dry Flo® Plus) |
| 1,0 | Gew.-% modifizierte Maisstärke (z. B. Mais PO4® PH"B") |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 9

| | |
|---|---|
| 1,0 | Gew.-% Ceteareth-20 (z. B. Eumulgin® B2) |
| 7,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Behenylalkohol (z. B. Lanette® 22) |
| 3,0 | Gew.-% Cocoglyceride (z. B. Novata® AB) |
| 8,0 | Gew.-% Decamethylcyclopentasiloxan (z. B. Dow Corning® DC345-Fluid) |
| 1,5 | Gew.-% Dimethicone (z. B. Baysilon® M 350) |
| 0,5 | Gew.-% Aluminium-Stärke-Octenylsuccinat (z. B. Dry Flo® Plus) |
| 1,5 | Gew.-% Cellulose (z. B. Vitacel® L 600-20 FCC) |
| 40 | Gew.-% Aluminiumchlorohydrat (50%-ige Lsg. in Wasser) |
| 0,6 | Gew.-% Parfümöl |
| 0,2 | Gew.-% Methylparaben |
| ad 100 Wasser VE | |

### Beispiel 10

| | |
|---|---|
| 1,0 | Gew.-% Ceteareth-20 (z. B. Eumulgin® B2) |
| 9,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Behenylalkohol (z. B. Lanette® 22) |
| 3,0 | Gew.-% Cocoglyceride (z. B. Novata® AB) |
| 8,0 | Gew.-% Decamethylcyclopentasiloxan (z. B. Dow Corning® DC345-Fluid) |
| 1,5 | Gew.-% Dimethicone (z. B. Baysilon® M 350) |
| 0,5 | Gew.-% Aluminium-Stärke-Octenylsuccinat (z. B. Dry Flo® Plus) |
| 1,5 | Gew.-% modifizierte Maisstärke (z. B. Mais PO4® PH"B") |
| 40 | Gew.-% Aluminiumchlorohydrat (50%-ige Lsg. in Wasser) |
| 0,6 | Gew.-% Parfümöl |
| 0,05 | Gew.% Propylgallat |
| ad 100 Gew.-% Wasser VE | |

### Beispiel 11

| | |
|---|---|
| 1,0 | Gew.-% Ceteareth-20 (z. B. Eumulgin® B2) |
| 9,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Behenylalkohol (z. B. Lanette® 22) |
| 1,0 | Gew.-% C₁₆-C₁₈-Fettsäure (z. B. Cutina® FS 45) |
| 3,0 | Gew.-% Cocoglyceride (z. B. Novata® AB) |
| 6,0 | Gew.-% Decamethylcyclopentasiloxan (z. B. Dow Coming® DC345-Fluid) |
| 1,5 | Gew.-% Dimethicone (z. B. Baysilon® M 350) |
| 1,0 | Gew.-% Aluminium-Stärke-Octenylsuccinat (z. B. Dry Flo® Plus) |
| 1,0 | Gew.-% modifizierte Reisstärke (z. B. P.F.A. 11) |
| 40 | Gew.-% Aluminiumchlorohydrat (50%-ige Lsg. in Wasser) |
| 0,6 | Gew.-% Parfümöl |
| 0,1 | Gew.-% Triclosan |
| ad 100 Gew.-% Wasser VE | |

### Beispiel 12

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Behenylalkohol (z. B. Lanette® 22) |
| 1,0 | Gew.-% C₁₆-C₁₈-Fettsäure (z. B. Cutina® FS 45) |
| 3,0 | Gew.-% Cocoglyceride (z. B. Novata® AB) |
| 8,0 | Gew.-% Decamethylcyclopentasiloxan (z. B. Dow Corning® DC345-Fluid) |
| 1,5 | Gew.-% Dimethicone (z. B. Baysilon® M 350) |
| 1,0 | Gew.-% Aluminium-Stärke-Octenylsuccinat (z. B. Dry Flo® Plus) |
| 1,0 | Gew.-% Cellulose (z. B. Vitacel® L 600-20 FCC) |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 13

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Gehärtete Palmölglyceride (z. B. Monomuls® 60-35C) |
| 1,5 | Gew.-% Behenylalkohol (z. B. Lanette® 22) |
| 3,0 | Gew.-% Cocoglyceride (z. B. Novata® AB) |
| 8,0 | Gew.-% Decamethylcyclopentasiloxan (z. B. Dow Corning® DC345-Fluid) |
| 1,5 | Gew.-% Dimethicone (z. B. Baysilon® M 350) |
| 1,5 | Gew.-% Aluminium-Stärke-Octenylsuccinat (z. B. Dry® Flo Plus) |
| 0,5 | Gew.-% Cellulose (z. B. Vitacel® L 600-20 FCC) |
| 4,0 | Gew.-% 1,2-Propylenglycol |
| 40 | Gew.-% Aluminiumchlorohydrat (50%-ige Lsg. in Wasser) |
| 0,6 | Gew.-% Parfümöl |
| 0,2 | Gew.-% Tocopherylacetat |
| 0,5 | Gew.-% Phenoxyethanol |
| ad 100 Gew.-% Wasser VE | |

### Beispiel 14

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Cetyl/Stearylalkohol (z. B. Stenol® 1618) |
| 3,0 | Gew.-% Cocoglyceride (z. B. Novata® AB) |
| 8,0 | Gew.-% Decamethylcyclopentasiloxan (z. B. Dow Corning® DC345-Fluid) |
| 1,5 | Gew.-% Dimethicone (z. B. Baysilon® M 350) |
| 1,5 | Gew.-% Aluminium-Stärke-Octenylsuccinat (z. B. Dry Flo® Plus) |
| 0,5 | Gew.-% Cellulose (z. B. Vitacel® L 600-20 FCC) |
| 40 | Gew.-% aktiviertes Aluminiumchlorohydrat (50%-ige Lsg. in Wasser, z. B. Reach® 501) |
| 0,6 | Gew.-% Parfümöl |
| ad 100 Gew.-% Wasser VE | |

### Beispiel 15

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Gehärtete Palmölglyceride (z. B. Monomuls® 60-35C) |
| 1,5 | Gew.-% Stearyl/Behenylalkohol (z. B. Stenol® 1822) |
| 3,0 | Gew.-% Cocoglyceride (z. B. Novata® AB) |
| 8,0 | Gew.-% Decamethylcyclopentasiloxan (z. B. Dow Corning® DC345-Fluid) |
| 1,5 | Gew.-% Dimethicone (z. B. Baysilon® M 350) |
| 1,5 | Gew.-% Aluminium-Stärke-Octenylsuccinat (z. B. Dry-Flo Plus® ) |
| 0,5 | Gew.-% modifizierte Reisstärke (z. B. P.F.A. 11) |
| 40 | Gew.-% Aluminiumsesquichlorohydrat (50%-ige Lsg. in Wasser, z. B. Reach® 301) |
| 0,6 | Gew.-% Parfümöl |
| 0,02 | Gew.-% Butylhydroxytoluol |
| ad 100 Gew.-% Wasser VE | |

### Beispiel 16

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Cetyl/Stearylalkohol (z. B. Stenol® 1618) |
| 3,0 | Gew.-% Cocoglyceride (z. B. Novata® A) |
| 8,0 | Gew.-% Decamethylcyclopentasiloxan (z. B. Dow Corning® DC245-Fluid) |
| 1,5 | Gew.-% Dimethicone (z. B. Baysilon® M 350) |
| 1,7 | Gew.-% Aluminium-Stärke-Octenylsuccinat (z. B. Dry Flo® Plus) |
| 0,3 | Gew.-% modifizierte Reisstärke (z. B. P.F.A. 11) |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 17

| | |
|---|---|
| 1,0 | Gew.-% Ceteareth-20 (z. B. Eumulgin® B2) |
| 6,0 | Gew.-% C₂₀-C₄₀-Alkylstearat (z. B. Kesterwachs® K82H) |
| 1,5 | Gew.-% Gehärtete Palmölglyceride (z. B. Monomuls® 60-35C) |
| 1,5 | Gew.-% Stearyl/Behenylalkohol (z. B. Stenol® 1822) |
| 3,0 | Gew.-% Cocoglyceride (z. B. Novata® A) |
| 8,0 | Gew.-% Decamethylcyclopentasiloxan (z. B. Dow Corning® DC245-Fluid) |
| 1,5 | Gew.-% Dimethicone (z. B. Baysilon® M 350) |
| 1,7 | Gew.-% Aluminium-Stärke-Octenylsuccinat (z. B. Dry Flo® Plus) |
| 0,3 | Gew.-% modifizierte Reisstärke (z. B. P.F.A. 11) |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 18

| | |
|---|---|
| 1 | Gew.-% Ceteareth-20 (z. B. Eumulgin® B2) |
| 5 | Gew.-% C₂₀-C₄₀-Alkylstearat (z. B. Kesterwachs® K82H) |
| 2 | Gew.-% Glycerinmonostearat (z. B. Cutina® GMS-V) |
| 2 | Gew.-% Behenylalkohol (z. B. Lanette® 22) |
| 3,0 | Gew.-% Cocoglyceride (z. B. Novata® AB) |
| 8,0 | Gew.-% Decamethylcyclopentasiloxan (z. B. Dow Corning® DC245-Fluid) |
| 1 | Gew.-% Dimethicone (z. B. Baysilon® M 350) |
| 1,7 | Gew.-% Aluminium-Stärke-Octenylsuccinat (z. B. Dry Flo® Plus) |
| 0,3 | Gew.-% Cellulose (z. B. Vitacel® L 600-20 FCC) |
| 4 | Gew.-% 1,2-Propylenglycol |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 19

| | |
|---|---|
| 1 | Gew.-% Ceteareth-30 (z. B. Eumulgin® B3) |
| 5 | Gew.-% C₂₀₋C₄₀-Alkylstearat (z. B. Kesterwachs® K82H) |
| 1,5 | Gew.-% Glycerinmonostearat (z. B. Cutina® GMS-V) |
| 1,5 | Gew.-% Behenylalkohol (z. B. Lanette® 22) |
| 3,0 | Gew.-% Cocoglyceride (z. B. Novata® AB) |
| 15,0 | Gew.-% Decamethylcyclopentasiloxan (z. B. Dow Corning® DC245-Fluid) |
| 1 | Gew.-% Dimethicone (z. B. Baysilon® M 350) |
| 1,8 | Gew.-% Aluminium-Stärke-Octenylsuccinat (z. B. Dry Flo® Plus) |
| 0,2 | Gew.-% Cellulose (z. B. Vitacel® L 600-20 FCC) |
| 4 | Gew.-% 1,2-Propylenglycol |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 20

| | |
|---|---|
| 0,5 | Gew.-% Ceteareth-20 (z. B. Eumulgin® B2) |
| 0,5 | Gew.-% Ceteareth-30 (z. B. Eumulgin® B3) |
| 6,0 | Gew.-% C₂₀-C₄₀-Alkylstearat (z. B. Kesterwachs® K82H) |
| 1,5 | Gew.-% Glycerylstearat (z. B. Cutina® GMS-V) |
| 1,5 | Gew.-% Cetyl/Stearylalkohol (z. B. Stenol® 1618) |
| 3,0 | Gew.-% Cocoglyceride (z. B. Novata® AB) |
| 8,0 | Gew.-% Decamethylcyclopentasiloxan (z. B. Dow Corning® DC245-Fluid) |
| 1,5 | Gew.-% Dimethicone (z. B. Baysilon® M 350) |
| 0,3 | Gew.-% Aluminium-Stärke-Octenylsuccinat (z. B. Dry Flo® Plus) |
| 1,7 | Gew.-% Cellulose (z. B. Vitacel® L 600-20 FCC) |
| 40 | Gew.-% Aluminiumchlorohydrat (50%-ige Lsg. in Wasser) |
| 0,6 | Gew.-% Parfümöl |
| 0,1 | Gew.-% Tocopherolacetat |
| ad 100 Gew.-% Wasser VE | |

### Beispiel 21

| | |
|---|---|
| 0,5 | Gew.-% Ceteareth-20 (z. B. Eumulgin® B2) |
| 0,5 | Gew.-% Ceteareth-30 (z. B. Eumulgin® B3) |
| 6,0 | Gew.-% C₂₀-C₄₀-Alkylstearat (z. B. Kesterwachs® K82H) |
| 1,5 | Gew.-% Gehärtete Palmölglyceride (z. B. Monomuls® 60-35C) |
| 1,5 | Gew.-% Cetyl/Stearylalkohol (z. B. Stenol® 1618) |
| 1,0 | Gew.-% C₁₆-C₁₈-Fettsäure (z. B. Cutina® FS 45) |
| 3,0 | Gew.-% Cocoglyceride (z. B. Novata® AB) |
| 8,0 | Gew.-% Decamethylcyclopentasiloxan (z. B. Dow Corning® DC245-Fluid) |
| 1,5 | Gew.-% Dimethicone (z. B. Baysilon® M 350) |
| 1,0 | Gew.-% Aluminium-Stärke-Octenylsuccinat (z. B. Dry Flo® Plus) |
| 1,0 | Gew.-% Cellulose (z. B. Vitacel® L 600-20 FCC) |
| 40 | Gew.-% Aluminiumchlorohydrat (50%-ige Lsg. in Wasser) |
| 0,6 | Gew.-% Parfümöl |
| 0,1 | Gew.-% Tocopherolacetat |
| ad 100 Gew.-% Wasser VE | |

### Beispiel 22

| | |
|---|---|
| 0,5 | Gew.-% Ceteareth-20 (z. B. Eumulgin® B2) |
| 0,5 | Gew.-% Ceteareth-30 (z. B. Eumulgin® B3) |
| 6,0 | Gew.-% C₂₀-C₄₀-Alkylstearat (z. B. Kesterwachs® K82H) |
| 1,5 | Gew.-% Gehärtete Palmölglyceride (z. B. Monomuls® 60-35C) |
| 1,5 | Gew.-% Cetyl/Stearylalkohol (z. B. Stenol® 1618) |
| 1,0 | Gew.-% C₁₆-C₁₈-Fettsäure (z. B. Cutina® FS 45) |
| 3,0 | Gew.-% Cocoglyceride (z. B. Novata® AB) |
| 8,0 | Gew.-% Decamethylcyclopentasiloxan (z. B. Dow Corning® DC245-Fluid) |
| 1,5 | Gew.-% Dimethicone (z. B. Baysilon® M 350) |
| 1,5 | Gew.-% Aluminium-Stärke-Octenylsuccinat (z. B. Dry Flo® Plus) |
| 0,5 | Gew.-% Cellulose (z. B. Vitacel® L 600-20 FCC) |
| 4 | Gew.-% 1,3-Butylenglycol |
| 40 | Gew.-% Aluminiumchlorohydrat (50%-ige Lsg. in Wasser) |
| 0,6 | Gew.-% Parfümöl |
| 0,1 | Gew.-% Tocopherolacetat |
| ad 100 Gew.-% Wasser VE | |

### Beispiel 23

| | |
|---|---|
| 1,5 | Gew.-% Ceteareth-20 (z. B. Eumulgin® B2) |
| 5,0 | Gew.-% C₂₀-C₄₀-Alkylstearat (z. B. Kesterwachs® K82H) |
| 1,0 | Gew.-% Glycerylstearat (z. B. Cutina® GMS-V) |
| 1,5 | Gew.-% Stearyl/Behenylalkohol (z. B. Stenol® 1822 A) |
| 0,5 | Gew.-% C₁₆-C₁₈-Fettsäure (z. B. Cutina® FS 45) |
| 2,0 | Gew.-% Cocoglyceride (z. B. Novata® AB) |
| 2,0 | Gew.-% Myristylmyristat (z. B. Cetiol® MM) |
| 4,0 | Gew.-% Hexyldecanol/Hexyldecyllaurat (z. B. Cetiol® PGL) |
| 4,0 | Gew.-% Decamethylcyclopentasiloxan (z. B. Dow Corning® DC245) |
| 2,0 | Gew.-% Aluminium-Stärke-Octenylsuccinat (z. B. Dry Flo® Plus) |
| 1,0 | Gew.-% modifizierte Reisstärke (z. B. Rice NS) |
| 0,2 | Gew.-% modifizierte Hydroxyethylcellulose (z. B. Natrosol Plus® 330) |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 24

| | |
|---|---|
| 1,0 | Gew.-% Ceteareth-20 (z. B. Eumulgin® B2) |
| 0,5 | Gew.-% Ceteareth-30 (z. B. Eumulgin® B3) |
| 5,0 | Gew.-% C₂₀-C₄₀-Alkylstearat (z. B. Kesterwachs® K82H) |
| 1,0 | Gew.-% Glycerylstearat (z. B. Cutina® GMS-V) |
| 1,5 | Gew.-% Stearyl/Behenylalkohol (z. B. Stenol® 1822 A) |
| 0,5 | Gew.-% C₁₆-C₁₈-Fettsäure (z. B. Cutina® FS 45) |
| 2,0 | Gew.-% Cocoglyceride (z. B. Novata® AB) |
| 3,0 | Gew.-% Myristylmyristat (z. B. Cetiol® MM) |
| 4,0 | Gew.-% Hexyldecanol/Hexyldecyllaurat (z. B. Cetiol® PGL) |
| 4,0 | Gew.-% Decamethylcyclopentasiloxan (z. B. Dow Corning® DC245) |
| 2,0 | Gew.-% Aluminium-Stärke-Octenylsuccinat (z. B. Dry Flo® Plus) |
| 1,0 | Gew.-% modifizierte Reisstärke (z. B. Rice NS) |
| 0,2 | Gew.-% modifizierte Hydroxyethylcellulose (z. B. Natrosol Plus® 330) |
| 40 | Gew.-% Aluminiumchlorohydrat (50%-ige Lsg. in Wasser) |
| 0,6 | Gew.-% Parfümöl |
| ad 100 Gew.-% Wasser VE | |

### Beispiel 25

| | |
|---|---|
| 1,0 | Gew.-% Ceteareth-20 (z. B. Eumulgin® B2) |
| 5,0 | Gew.-% C₂₀-C₄₀-Alkylstearat (z. B. Kesterwachs® K82H) |
| 1,0 | Gew.-% Glycerylstearat (z. B. Cutina® GMS-V) |
| 1,5 | Gew.-% Stearyl/Behenylalkohol (z. B. Stenol® 1822 A) |
| 3,0 | Gew.-% Cocoglyceride (z. B. Novata® AB) |
| 3,0 | Gew.-% Myristylmyristat (z. B. Cetiol® MM) |
| 4,0 | Gew.-% Hexyldecanol/Hexyldecyllaurat (z. B. Cetiol® PGL) |
| 4,0 | Gew.-% Decamethylcylopentasiloxan (z. B. Dow Corning® DC245-Fluid) |
| 2,0 | Gew.-% Aluminium-Stärke-Octenylsuccinat (z. B. Dry Flo® Plus) |
| 1,0 | Gew.-% modifizierte Reisstärke (z. B. Rice NS) |
| 0,2 | Gew.-% Hydroxyethylcellulose (z. B. Natrosol Plus® 250 HHX) |
| 40 | Gew.-% Alumniniumchlorohydrat (50%-ige Lsg. in Wasser) |
| 0,6 | Gew.-% Parfümöl |
| ad 100 Gew.-% Wasser VE | |

### Beispiel 26

| | |
|---|---|
| 1,0 | Gew.-% Ceteareth-20 (z. B. Eumulgin® B2) |
| 5,0 | Gew.-% C₂₀-C₄₀- Alkylstearat (z. B. Kesterwachs® K82H) |
| 1,0 | Gew.-% Glycerylstearat (z. B. Cutina® GMS-V) |
| 1,5 | Gew.-% Stearyl/Behenylalkohol (z. B. Stenol® 1822 A) |
| 3,0 | Gew.-% Cocoglyceride (z. B. Novata® AB) |
| 3,0 | Gew.-% Myristylmyristat (z. B. Cetiol® MM) |
| 4,0 | Gew.-% Hexyldecanol/Hexyldecyllaurat (z. B. Cetiol® PGL) |
| 4,0 | Gew.-% Decamethylcylopentasiloxan (z. B. Dow Corning® DC245-Fluid) |
| 2,0 | Gew.-% Aluminium-Stärke-Octenylsuccinat (z. B. Dry Flo® Plus) |
| 1,0 | Gew.-% modifizierte Reisstärke (z. B. Rice NS) |
| 0,2 | Gew.-% Methylhydroxypropylcellulose (z. B. Culminal® MHPC 3000) |
| 40 | Gew.-% Alumniniumchlorohydrat (50%-ige Lsg. in Wasser) |
| 0,6 | Gew.-% Parfümöl |
| ad 100 Gew.-% Wasser VE | |

### Beispiel 27

| | |
|---|---|
| 1,0 | Gew.-% Ceteareth-20 (z. B. Eumulgin® B2) |
| 5,0 | Gew.-% C₂₀-C₄₀-Alkylstearat (z. B. Kesterwachs® K82H) |
| 1,0 | Gew.-% Glycerylstearat (z. B. Cutina® GMS-V) |
| 1,5 | Gew.-% Stearyl/Behenylalkohol (z. B. Stenol® 1822 A) |
| 1,0 | Gew.-% C₁₆-C₁₈-Fettsäure (z. B. Cutina® FS 45) |
| 2,0 | Gew.-% Cocoglyceride (z. B. Novata® AB) |
| 3,0 | Gew.-% Cetylstearylstearat (z. B. Crodamol® CSS) |
| 4,0 | Gew.-% Hexyldecano/Hexyldecyllaurat (z. B. Cetiol® PGL) |
| 4,0 | Gew.-% Decamethylcylopentasiloxan (z. B. Dow Corning® DC245-Fluid) |
| 2,0 | Gew.-% Aluminium-Stärke-Octenylsuccinat (z. B. Dry Flo® Plus) |
| 1,0 | Gew.-% modifizierte Reisstärke (z. B. Rice NS) |
| 40 | Gew.-% Alumniniumchlorohydrat (50%-ige Lösung in Wasser) |
| 0,6 | Gew.-% Parfümöl |
| ad 100 Gew.-% Wasser VE | |

### Vergleichsbeispiel (nicht erfindungsgemäß)

| | |
|---|---|
| 1 | Gew.-% Behenylalkohol + 10 EO (z. B. Mergital® B10) |
| 5 | Gew.-% C₂₀-C₄₀-Alkylstearat (Kesterwachs® K82H) |
| 1,5 | Gew.-%Triglycerindiisostearat (z. B. Lameform® TGI) |
| 10 | Gew.-% Decamethylcyclopentasiloxan (z. B. Dow Corning® 345-Fluid) |
| 1,5 | Gew.-% Polydimethylsiloxan (z. B. Baysilon® M350) |
| 3 | Gew.-% Silikonöl (z. B. Dow Coming DC® 200 20cS) |
| 10. | Gew.-% Aluminium-Stärke-Octenylsuccinat (z. B. Dry Flo® Plus) |
| 2 | Gew.-% Talkum |
| 4 | Gew.-% 1,3-Butylenglycol |
| 40 | Gew.-% Aluminiumchlorohydrat (50%-ige Lsg. in Wasser) |
| 1 | Gew.-% Parfümöl |
| ad 100 Gew.-% Wasser VE | |

Alle erfindungsgemäßen Formulierungen hinterließen beim Anwender ein trockenes, samtiges Hautgefühl, einen angenehm kühlenden Effekt und ziehen schnell ein. Die Rezeptur des Vergleichsbeispiels, die Talkum anstelle der Cellulose enthält, hinterläßt mehr sichtbare Rückstände und wird von den Anwendem als sensorisch weniger befriedigend beurteilt.

### Anhang

1) Baysilon® M350
   INCI: Dimethicone
   Hersteller: Bayer
2) Cetiol® PGL
   INCI: Hexyldecanol, Hexydecyl laurate
   Hersteller: Cognis Deutschland GmbH (Henkel)
3) Cetiol® MM
   INCI: Myristyl myristate
   Hersteller: Cognis Deutschland GmbH (Henkel)
4) Chlorhydrol® 50 w/w-Lösung
   INCI: Aluminium chlorohydrate (50%-ige Lösung in Wasser)
   Hersteller: Reheis
5) Crodamol® CCS
   INCI: Cetearyl stearate
   Hersteller: Croda
6) Culminal® MHPC 3000
   INCI: Hydroxypropyl methylcellulose
   Hersteller: Hercules
7) Cutina® GMS-V
   INCI: Glyceryl stearate
   Hersteller: Cognis Deutschland GmbH (Henkel)
8) Cutina® FS45
   INCI: Palmitic acid, stearic acid
   Hersteller: Cognis Deutschland GmbH (Henkel)
9) Dow Corning® DC245
   INCI: Cyclomethicone
   Hersteller: Dow Corning
10) Dow Corning® DC345
   INCI: Cyclomethicone
   Hersteller: Dow Corning
11) Dow Coming DC® 200 20 cS
   INCI: Dimethicone
   Hersteller: Dow Corning
12) Dry Flo® Plus
   INCI: Aluminium octenyl succinate
   Hersteller: National starch
13) Eumulgin® B2
   INCI: Ceteareth-20
   Hersteller: Cognis Deutschland GmbH (Henkel)
14) Eumulgin® B3
   INCI: Ceteareth-30
   Hersteller: Cognis Deutschland GmbH (Henkel)
15) Kesterwachs® K82H
   INCI: C₂₀-C₄₀-Alkystearate
   Hersteller: Koster Keunen Holland bv
16) Lameform® TGI
   INCI: Polyglyceryl-3-Diisostearate
   Hersteller: Henkel (Grünau)
17) Lanette® 22
   INCI: Behenyl alcohol
   Hersteller: Cognis France S.A.
18) Mais PO4® PH"B"
   Modifizierte Maisstärke
   Hersteller: Dr. Hauser GmbH
19) Mergital® B10
   INCI: Beheneth-10
   Hersteller: Henkel (Sidobre-Sinnova)
20) Monomuls® 60-35C
   INCI: Hydrogenated palm glycerides
   Hersteller: Henkel (Grünau)
21) Monomuls® 90-O-18
   INCI: Glyceryl oleate
   Hersteller: Henkel (Grünau)
22) Natrosol Plus® 330
   Hydroxyethylcellulose-Derivat
   Hersteller: Hercules
23)Natrosol Plus® 250 HHX
   Hydroxyethylcellulose
   Hersteller: Hercules
24) Novata® A
   INCI: Cocoglycerides
   Hersteller: Henkel
25) Novata® AB
   INCI: Cocoglycerides
   Hersteller: Henkel
26) Optigel® SH
   Magnesiumsilikat
   Hersteller: Süd Chemie
27) P.F.A. 11
   Modifizierte Reisstärke
   Hersteller: Dr. Hauser GmbH
28) Reach® 301 Solution
   INCI: Aluminium sesquichlorohydrate (50%-ige Lösung in Wasser)
   Hersteller: Reheis
29) Reach® 501 Solution
   INCI: Aluminium chlorohydrate (50%-ige Lösung in Wasser)
   Hersteller: Reheis
30) Rezal® 36G
   Aluminium-Zirkonium-Tetrachlorohydrex-Glycin, 35%- ige Lösung in Wasser
   Hersteller: Reheis Inc.
31) Rice NS
   Modifizierte Reisstärke
   Hersteller: Dr. Hauser GmbH
32) Stenol® 1618
   INCI: Cetearyl alcohol
   Hersteller: Cognis Deutschland GmbH
33) Stenol® 1822
   INCI: Behenyl alcohol
   Hersteller: Cognis France S.A.
34) Vitacel® L 600/20 FCC
   Cellulosepulver
   Hersteller: J. Rettenmaier & Söhne

## Patentansprüche

1. Wasserhaltige Antitranspirant-Zusammensetzung, **dadurch gekennzeichnet daß** sie
a) wenigstens zwei teilchenförmige, wasserunlösliche Polysaccharide und/oder Polysaccharid-Derivate
b) wenigstens einen, in Wasser gelösten, adstringierenden Antitranspirant-Wirkstoff und
c) wenigstens eine Wachskomponente
enthält.

2. Zusammensetzung gemäß Anspruch 1, **dadurch gekennzeichnet, daß** die Polysaccharide ausgewählt sind aus der Gruppe der Stärke und Cellulose.

3. Zusammensetzung gemäß Anspruch 2, **dadurch gekennzeichnet, daß** die Stärke hydrophob modifiziert ist.

4. Zusammensetzung gemäß einem der Ansprüche 1 bis 3, **dadurch gekennzeichnet, daß** der Antitranspirant-Wirkstoff ein adstringierendes Aluminiumsalz ist.

5. Zusammensetzung gemäß einem der Ansprüche 1 bis 4, **dadurch gekennzeichnet, daß** die Wachskomponente ein Ester aus einem gesättigten, einwertigen C₂₀-C₆₀-Alkohol und einer gesättigten C₈-C₃₀-Monocarbonsäure ist.

6. Zusammensetzung gemäß einem der Ansprüche 1 bis 5, **dadurch gekennzeichnet, daß** sie zusätzlich wenigstens einen nicht-ionischen Emulgator mit einem HLB-Wert von 8-18 enthält.

7. Zusammensetzung nach Anspruch 6, **dadurch gekennzeichnet, daß** sie wenigstens einen lipophilen Coemulgator enthält.

8. Zusammensetzung gemäß einem der Ansprüche 1 bis 7, **dadurch gekennzeichnet, daß** sie eine ÖI-in-Wasser-Dispersion ist.

9. Zusammensetzung gemäß einem der Ansprüche 1 bis 8, **dadurch gekennzeichnet, daß** sie zusätzlich wenigstens eine flüchtige Silikonverbindung enthält.

10. Zusammensetzung gemäß einem der Ansprüche 1 bis 9, **dadurch gekennzeichnet, daß** sie bei 25°C eine Viskosität von mindestens 80000 mPa·s aufweist.

11. Zusammensetzung gemäß einem der Ansprüche 1 bis 10, **dadurch gekennzeichnet, daß** sie zusätzlich wenigstens ein Silikat enthält.

12. Zusammensetzung gemäß einem der Ansprüche 1 bis 11, **dadurch gekennzeichnet, daß** sie zusätzlich wenigstens einen Glycerinester mit einem Schmelzpunkt im Bereich von 28 - 45° C enthält.

13. Zusammensetzung gemäß einem der Ansprüche 1 bis 12, **dadurch gekennzeichnet, daß** sie wenigstens folgende Komponenten enthält:
a) 0,1 - 25 Gew.-% einer teilchenförmigen Stärke
b) 0,1 - 25 Gew.-% einer teilchenförmigen Cellulose
c) 0,1-15 Gew.-% eines Esters eines Fettalkohols mit einer Fettsäure
d) 0,1 - 40 Gew.-% eines adstringierenden Antitranspirantsalzes
e) 0,1 - 20 Gew.-% einer flüchtigen Silikonverbindung
f) 0,1 - 5 Gew.-% eines Emulgators mit einem HLB-Wert von 8 - 18
g) 10 - 65 Gew.-% Wasser

14. Verwendung einer Zusammensetzung gemäß einem der Ansprüche 1 bis 13 als schweißhemmendes Mittel zur Applikation auf der Haut.

15. Verfahren zur Herstellung einer Zusammensetzung gemäß einem der Ansprüche 1 bis 13, **dadurch gekennzeichnet, daß** die Wachs- und Ölkomponenten zusammen mit den Emulgatoren und Polysacchariden auf 90 - 95°C erwärmt und aufgeschmolzen werden, danach das Wasser mit den wasserlöslichen Wirkstoffen zugegeben wird und man die Masse durch Abkühlen auf Raumtemperatur verfestigt.

## Claims

1. A water-free antiperspirant composition, **characterized in that** it contains:
a) at least two particulate, water-insoluble polysaccharides and/or polysaccharide derivatives,
b) at least one astringent antiperspirant component dissolved in water and
c) at least one wax component.

2. A composition as claimed in claim 1, **characterized in that** the polysaccharides are selected from the group consisting of starch and cellulose.

3. A composition as claimed in claim 2, **characterized in that** the starch is hydrophobically modified.

4. A composition as claimed in any of claims 1 to 3, **characterized in that** the antiperspirant component is an astringent aluminium salt.

5. A composition as claimed in any of claims 1 to 4, **characterized in that** the wax component is an ester of a saturated, monohydric C₂₀₋₆₀ alcohol and a saturated C₈₋₃₀ monocarboxylic acid.

6. A composition as claimed in any of claims 1 to 5, **characterized in that** it additionally contains at least one nonionic emulsifier with an HLB value of 8 to 18.

7. A composition as claimed in claim 6, **characterized in that** it contains at least one lipophilic co-emulsifier.

8. A composition as claimed in any of claims 1 to 7, **characterized in that** it is an oil-in-water dispersion.

9. A composition as claimed in any of claims 1 to 8, **characterized in that** it additionally contains at least one volatile silicone compound.

10. A composition as claimed in any of claims 1 to 9, **characterized in that** it has a viscosity at 25°C of at least 80,000 mPa.s.

11. A composition as claimed in any of claims 1 to 10, **characterized in that** it additionally contains at least one silicate.

12. A composition as claimed in any of claims 1 to 11, **characterized in that** it additionally contains at least one glycerol ester with a melting point in the range of 28 to 45°C.

13. A composition as claimed in any of claims 1 to 12, **characterized in that** it contains at least the following components:
a) 0.1 to 25% by weight of a particulate starch,
b) 0.1 to 25% by weight of a particulate cellulose,
c) 0.1 to 15% by weight of an ester of a fatty alcohol with a fatty acid,
d) 0.1 to 40% by weight of an astringent antiperspirant salt,
e) 0.1 to 20% by weight of a volatile silicone compound,
f) 0.1 to 5% by weight of an emulsifier with an HLB value of 8 to 18,
g) 10 to 65% by weight of water.

14. The use of the composition claimed in any of claims 1 to 13 as a perspiration inhibitor for application to the skin

15. A process for the production of the composition claimed in any of claims 1 to 13, **characterized in that** the wax and oil components are heated to 90-95°C and melted together with the emulsifiers and polysaccharides, after which the water is added with the water-soluble components and the whole is solidified by cooling to room temperature.

## Revendications

1. Composition antisudérale contenant de l'eau **caractérisée en ce qu'**elle contient
a) au moins deux polysaccharides et/ou dérivés de polysaccharides particulaires insolubles dans l'eau
b) au moins une substance active antisudérale astringente dissoute dans l'eau et
c) au moins un composant de cire.

2. Composition selon la revendication 1, **caractérisée en ce que** les polysaccharides sont choisis parmi le groupe comprenant des amidons et de la cellulose.

3. Composition selon la revendication 2, **caractérisée en ce que** l'amidon a été soumis à une modification pour le rendre hydrophobe.

4. Composition selon l'une quelconque des revendications 1 à 3, **caractérisée en ce que** la substance active antisudérale est un sel d'aluminium astringent.

5. Composition selon l'une quelconque des revendications 1 à 4, **caractérisée en ce que** le composant de cire est un ester constitué par un alcool saturé monovalent en C₂₀-C₆₀ et par un acide monocarboxylique saturé en C₈-C₃₀.

6. Composition selon l'une quelconque des revendications 1 à 5, **caractérisée en ce qu'**elle contient en outre, au moins un émulsifiant non ionique possédant une valeur HLB de 8 à 18.

7. Composition selon la revendication 6, **caractérisée en ce qu'**elle contient au moins un coémulsifiant lipophile.

8. Composition selon l'une quelconque des revendications 1 à 7, **caractérisée en ce qu'**il s'agit d'une dispersion du type huile-dans-l'eau.

9. Composition selon l'une quelconque des revendications 1 à 8, **caractérisée en ce qu'**elle contient en outre au moins un composé de silicone volatil.

10. Composition selon l'une quelconque des revendications 1 à 9, **caractérisée en ce qu'**elle présente une viscosité à 25 °C d'au moins 80.000 mPa.s.

11. Composition selon l'une quelconque des revendications 1 à 10, **caractérisée en ce qu'**elle contient en outre au moins un silicate.

12. Composition selon l'une quelconque des revendications 1 à 11, **caractérisée en ce qu'**elle contient en outre au moins un ester glycérique possédant un point de fusion dans la plage de 28 à 45 °C.

13. Composition selon l'une quelconque des revendications 1 à 12, **caractérisée en ce qu'**elle contient au moins les composants ci-après :
a) à concurrence de 0,1 à 25 % en poids, un amidon particulaire
b) à concurrence de 0,1 à 25 % en poids, une cellulose particulaire
c) à concurrence de 0,1 à 15 % en poids, un ester d'un alcool gras avec un acide gras
d) à concurrence de 0,1 à 40 % en poids, un sel astringent antisudéral
e) à concurrence de 0,1 à 20 % en poids, un composé de silicone volatil
f) à concurrence de 0,1 à 5 % en poids, un émulsifiant possédant une valeur HLB de 8 à 18
g) à concurrence de 10 à 65 % en poids, de l'eau.

14. Utilisation d'une composition selon l'une quelconque des revendications 1 à 13, à titre d'agent inhibant la sudation à appliquer sur la peau.

15. Procédé pour la préparation d'une composition selon l'une quelconque des revendications 1 à 13, **caractérisé en ce qu'**on chauffe à une température de 90 à 95 °C et on porte à fusion les composants de cire et d'huile de manière conjointe avec les émulsifiants et les polysaccharides, ensuite on ajoute l'eau avec les substances actives hydrosolubles et on solidifie la masse par refroidissement à la température ambiante.
